(19) Europäisches Patentamt
European Patent Office
Office européen des brevets

(11) **EP 1 392 212 B2**

(12) **NEW EUROPEAN PATENT SPECIFICATION**
After opposition procedure

(45) Date of publication and mention
of the opposition decision:
**17.04.2013 Bulletin 2013/16**

(45) Mention of the grant of the patent:
**03.10.2007 Bulletin 2007/40**

(21) Application number: **02731763.5**

(22) Date of filing: **10.05.2002**

(51) Int Cl.:
*A61F 13/515* (2006.01)     *A61F 13/514* (2006.01)
*A61F 13/494* (2006.01)

(86) International application number:
**PCT/US2002/015014**

(87) International publication number:
**WO 2002/091974 (21.11.2002 Gazette 2002/47)**

(54) **ABSORBENT ARTICLE HAVING A BODY CONFORMING ABSORBENT COMPOSITE**

ABSORBIERENDER ARTIKEL MIT EINEM SICH DEM KÖRPER ANPASSENDEN
ABSORBIERENDEN ZWISCHENSTÜCK

ARTICLE ABSORBANT COMPRENANT UN COMPOSITE ABSORBANT QUI EPOUSE LA FORME
DU CORPS

(84) Designated Contracting States:
**DE FR GB IT NL SE**

(30) Priority: **14.05.2001 US 855200**

(43) Date of publication of application:
**03.03.2004 Bulletin 2004/10**

(73) Proprietor: **KIMBERLY-CLARK WORLDWIDE, INC.**
**Neenah, WI 54956 (US)**

(72) Inventors:
• **PRICE, Cindy, L.**
**Appleton, WI 54915 (US)**
• **GROSS, Jacqueline, A.**
**Neenah, WI 54956 (US)**

• **VAN GOMPEL, Paul, T.**
**Hortonville, WI 54944 (US)**

(74) Representative: **Mabey, Katherine Frances et al**
**Dehns**
**St Bride's House**
**10 Salisbury Square**
**London EC4Y 8JD (GB)**

(56) References cited:
| EP-A2- 0 630 632 | WO-A-96/14815 |
| WO-A-99/20215 | DE-A- 19 813 334 |
| DE-C2- 19 735 303 | GB-A- 2 253 131 |
| GB-A- 2 284 741 | US-A- 4 704 115 |
| US-A- 5 476 458 | US-A- 5 476 458 |
| US-A- 5 904 673 | |

## Description

## BACKGROUND

The present invention relates generally to an absorbent garment, and in particular, to an absorbent garment having a body conforming absorbent composite.

Absorbent garments, and in particular disposable absorbent garments, often include an absorbent composite and one or more body panels connected to the absorbent composite. Typically, the absorbent composites are secured to the body panels along the peripheral side edges of the absorbent composite. As such, the absorbent composite can reduce or otherwise affect the extensibility of the body panel, especially when it is desired to have a wider absorbent composite. In addition, the absorbent composite typically is not able to conform to the body of the user independently of the body panels, and can therefore distort the panels when fitted to a user.

In other embodiments, the absorbent garment may include a top sheet and backsheet, one or both of which can form in part longitudinally extending flaps. Typically, such flaps are directed inboard to form a trough and may have one or more ends thereof secured to the garment to prevent the inversion thereof. As such, the flaps do not contribute to the overall width of the absorbent composite.

WO 96/14815 discloses an absorbent article having inflected barriers cuffs.

## SUMMARY

The present invention provides an absorbent article in accordance with claim 1.

Briefly stated, in one aspect, the invention is directed to an absorbent garment comprising a body panel having a bodyside surface and an absorbent composite having a longitudinally extending length and a laterally extending width and comprising a backsheet, a topsheet and a retention portion disposed between the backsheet and the topsheet. The absorbent composite is connected to the bodyside surface of the body panel and comprises a side margin that is not attached to the body panel and that extends laterally outboard and terminates in a free edge. Preferably, die absorbent composite includes laterally opposed side margins.

In one preferred embodiment, the side margin is formed from the top sheet. In another preferred embodiment, the side margin is formed from the topsheet and the backsheet. In one preferred embodiment, a first portion of the topsheet is folded over a second portion of the topsheet to form a folded edge that defines the free edge of the side margin. In yet another preferred embodiment, the backsheet is disposed between the first and second portions of the topsheet. Portions of the side margins can include an elastic element.

In one preferred embodiment, the body panel comprises a first and second longitudinally spaced body panels. In yet another preferred embodiment, each of the first and second body panels includes a pair of laterally spaced side body panels.

In another aspect of the invention, a method of protecting a user from bodily exudates comprises applying the absorbent garment to the body of the user.

The present invention provides significant advantages over other absorbent garments and methods for the use and manufacture thereof. For example, the unattached side margins can fold inward and follow the bodylines of the user without distorting the body panels. Moreover, the unattached side margins do not restrict the fit of the leg regions of the panels during use. In addition, the absorbent capacity of the garment can be easily changed simply by increasing the width of the absorbent composite, while at the same time maintaining the width of the portion of the absorbent composite that is connected to the body panels. As such, the width of the side margins can be increased without affecting the overall fit of the garment.

The present invention, together with further objects and advantages, will be best understood by reference to the following detailed description taken in conjunction with the accompanying drawings.

## BRIEF DESCRIPTION OF THE DRAWINGS

Many of the features and dimensions portrayed in the drawings, and in particular the presentation of layer thicknesses and the like, have been somewhat exaggerated for the sake of illustration and clarity.

FIGURE 1 is a plan view of a first embodiment of an absorbent garment taken from the bodyside thereof.

FIGURE 2 is a schematic illustration of a cross-sectional view of the absorbent garment taken along line 2-2 in Figure 1.

FIGURE 3 is a schematic illustration of a cross-sectional view of the absorbent garment taken along line 3-3 in Figure 1.

FIGURE 4 is a schematic illustration of a cross-sectional view of the absorbent garment taken along line 4-4 in Figure 1.

FIGURE 5 is a plan view of a second embodiment of an absorbent garment taken from the bodyside thereof.

FIGURE 6 is a schematic illustration of a cross-sectional view of the absorbent garment taken along line 6-6 in Figure 5.

FIGURE 7 is a schematic illustration of a cross-sectional view of the absorbent garment taken along line 7-7 in Figure 5.

FIGURE 8 is a schematic illustration of a cross-sectional view of the absorbent garment taken along line 8-8 in Figure 5.

FIGURE 9 is a plan view of a third embodiment of an absorbent garment taken from the bodyside thereof.

FIGURE 10 is a schematic illustration of a cross-sectional view of the absorbent garment taken along line 10-10 in Figure 9.

FIGURE 11 is a schematic illustration of a cross-sectional view of the absorbent garment taken along line 11-11 in Figure 9.

FIGURE 12 is a schematic illustration of a cross-sectional view of the absorbent garment taken along line 12-12 in Figure 9.

FIGURE 13 is a plan view of a fourth embodiment of an absorbent garment taken from the bodyside thereof.

FIGURE 14 is a schematic illustration of a cross-sectional view of the absorbent garment taken along line 14-14 in Figure 13.

FIGURE 15 is a schematic illustration of a cross-sectional view of the absorbent garment taken along line 15-15 in Figure 13.

FIGURE 16 is a schematic illustration of a cross-sectional view of the absorbent garment taken along line 16-16 in Figure 13.

FIGURE 17 is a plan view of a fifth embodiment of an absorbent garment taken from the bodyside thereof.

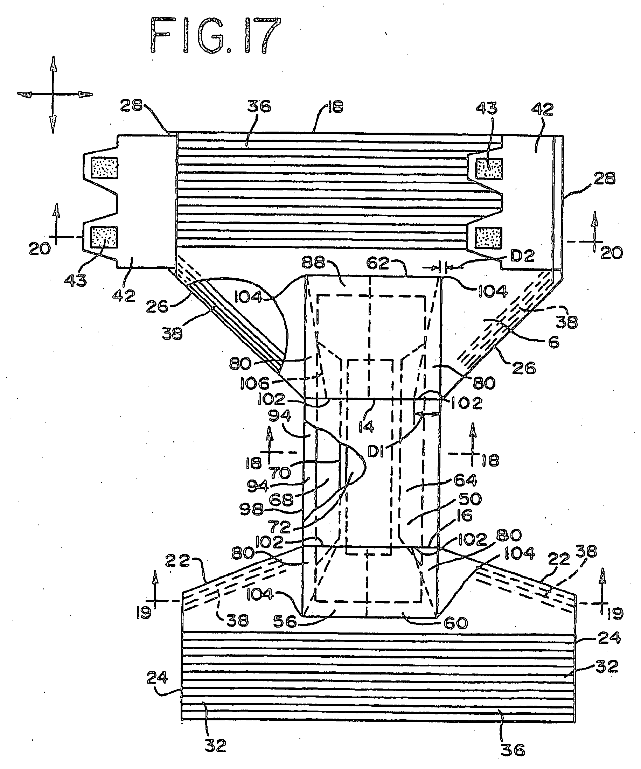

FIGURE-18 is a schematic illustration of a cross-sectional view of the absorbent garment taken along line 18-18 in Figure 17.

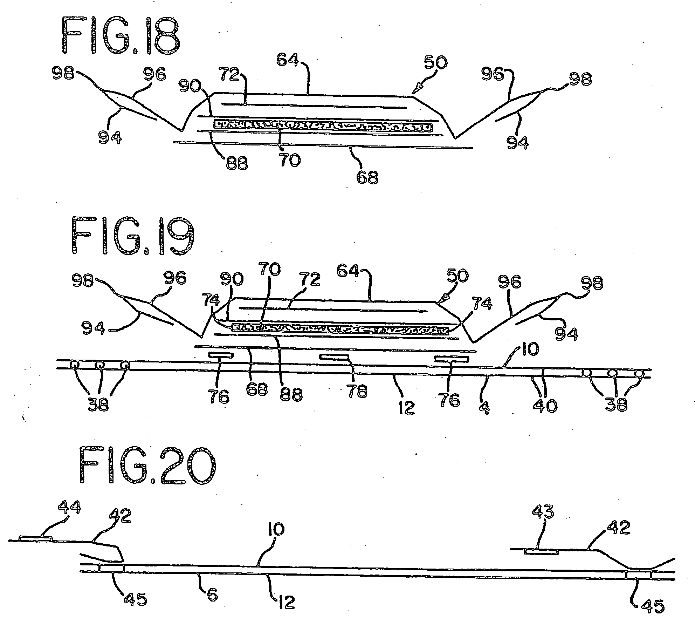

FIGURE 19 is a schematic illustration of a cross-sectional view of the absorbent garment taken along line 19-19 in Figure 17.

FIGURE 20 is a schematic illustration of a cross-sectional view of the absorbent garment taken along line 20-20 in Figure 17.

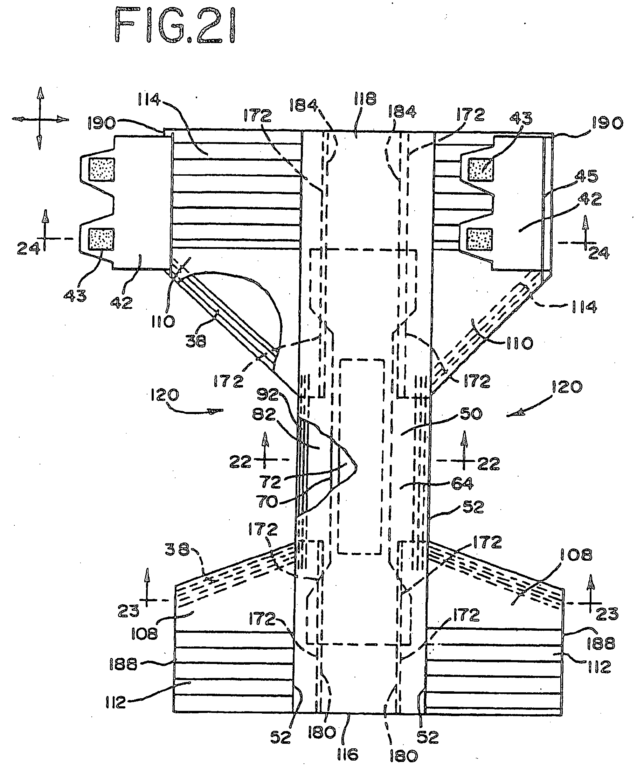

Figures 21 to 28 illustrate same further arrangements not in accordance with the invention, and which have been retained for the purposes of facilitating understanding of certain features of the invention.

FIGURE 21 is a plan view of an absorbent garment taken from the bodyside thereof.

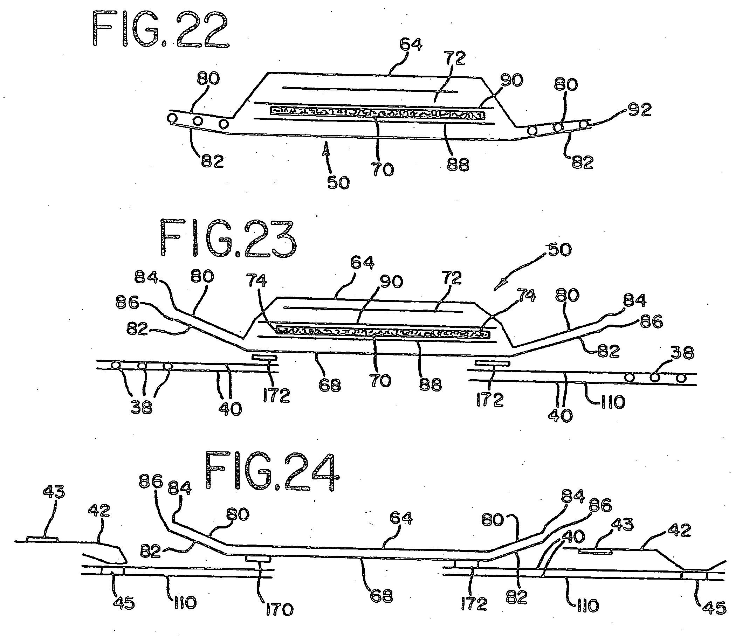

FIGURE 22 is a schematic illustration of a cross-sectional view of the absorbent garment taken along line 22-22 in Figure 21.

FIGURE 23 is a schematic illustration of a cross-sectional view of the absorbent garment taken along line 23-23 in Figure 21.

FIGURE 24 is a schematic illustration of a cross-sectional view of the absorbent garment taken along line 24-24 in Figure 21.

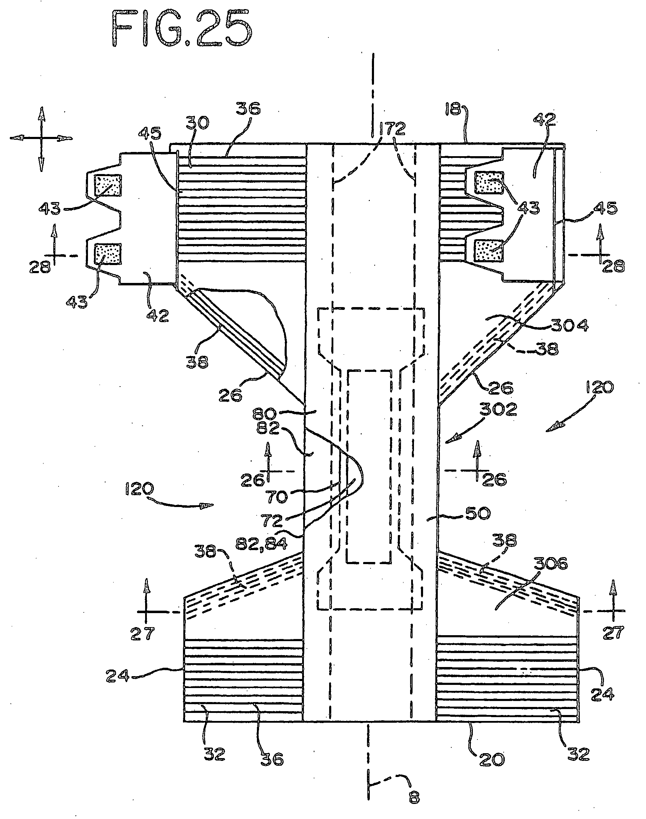

**FIGURE 25** is a plan view of an absorbent garment taken from the bodyside thereof.

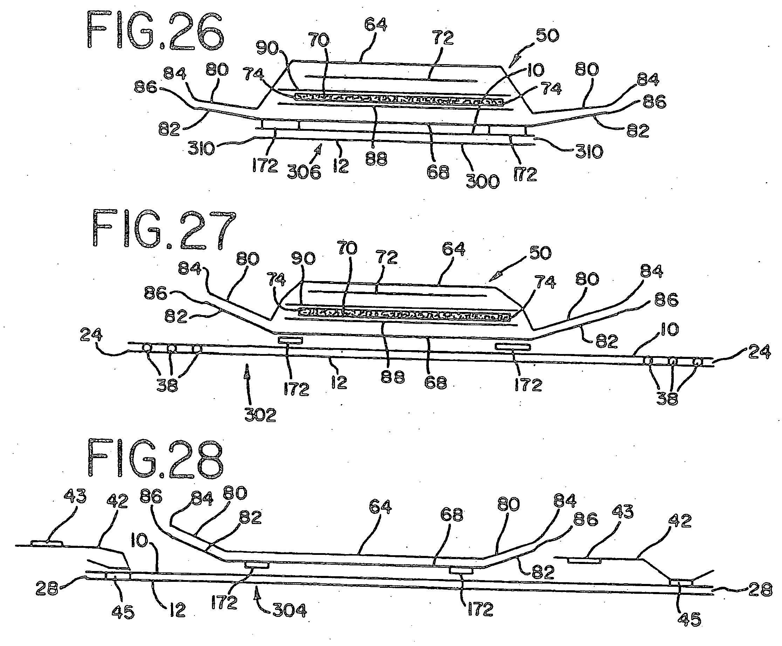

FIGURE 26 is a schematic illustration of a cross-sectional view of the absorbent garment taken along line 26-26 in Figure 25.

FIGURE 27 is a schematic illustration of a cross-sectional view of the absorbent garment taken along line 27-27 in Figure 25.

FIGURE 28 is a schematic illustration of a cross-sectional view of the absorbent garment taken along line 28-28 in Figure 25.

## DETAILED DESCRIPTION OF THE PRESENTLY PREFERRED EMBODIMENTS

It should be understood that the term "longitudinal," as used herein, means of or relating to length or the lengthwise direction, and in particular, the direction running between the front and back of the user. The term "laterally," as used herein means situated on, directed toward or running from side to side, and in particular, a direction running from the left to the right of a user. The terms "upper," "lower," "inner", and "outer" as used herein are intended to indicate the direction relative to the user wearing an absorbent garment over the crotch region, while the terms "inboard" and "outboard" refer to the directions relative to a centerline **8** of the garment. For example, the terms "inner" and "upper" refer to a "bodyside." which means the side closest to the body of the user, while the terms "outer" and "lower" refer to a "garment side". The term "bodyside" should not be interpreted to mean in contact with the body of the user, but rather simply means the side that would face toward the body of the user, regardless of whether the absorbent garment is actually being worn by the user and regardless of whether there are or may be intervening layers between the component and the body of the user. Likewise, the term "garment side" should not be interpreted to mean in contact with the garments of the user, but rather simply means the side that faces away from the body of the user, and therefore toward any outer garments that may be worn by the user, regardless of whether the absorbent garment is actually being worn by a user, regardless of whether any such outer garments are actually worn and regardless of whether there may be intervening layers between the component and any outer garment.

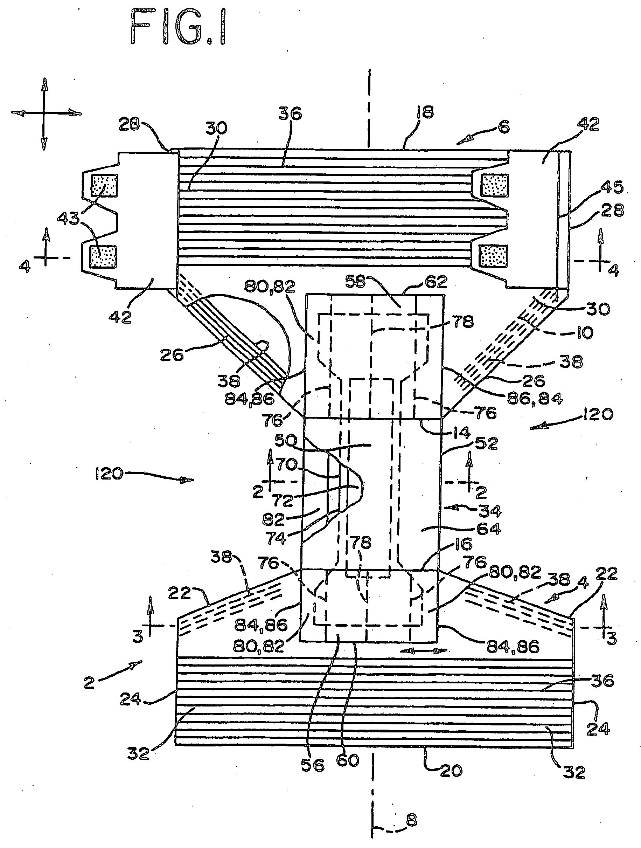

Referring to FIGS. 1, 5, 9, 13 and 17, an absorbent garment **2** includes a first, front body panel **4** and a second, rear body panel **6.** The first and second body panels each have an inner, bodyside surface **10** an outer, garment side surface and a length, which is less the overall length of the absorbent garment. Each of the first and second body panels has a first and second longitudinally opposed terminal end edges **16, 14, 20, 18,** and outer side edges, including a tapered edge **22, 26 and an** outboard edge **24, 28** formed along the outer periphery of laterally opposed ear portions **30, 32.** The first terminal edges **14, 16** of the first and second body panels are longitudinally spaced to form an opening 34 therebetween in the crotch region of the garment, while the second terminal edges **20, 18** of the first and second body panels form front and back waist edges respectively. A plurality, meaning two or more, of laterally extending elastic elements **36** can be secured to each of the first and second body panels. Likewise, one or more leg elastic elements **38** can be secured along the tapered side edge of the body panels to form a gasket with the leg of the user. For example, as shown in FIGS. 1-4, each panel can be made of an elasticized composite panel material comprising two non-woven substrates **40** with the plurality of elastic strands **38, 36** sandwiched therebetween. The elastic strands are positioned in the waist regions and along the leg perimeters. A portion of the leg elastic elements **38** can extend under a side margin of an absorbent composite **50**. The placement of the panel leg elastic elements further inward along the side edge provides for improved fit and performance of the garment.

In an alternative arrangement, shown in FIG. 21, an absorbent composite **50** extends longitudinally along the entire extent of the garment from one end **116** to the other end **118** thereof. This arrangement is not in accordance with the invention, but has been retained for the purposes of facilitating understanding of certain features of the invention. A pair of front, side body panels **108** have inboard edges **180** that are secured to opposite side regions of the absorbent composite, preferably on the bodyside thereof, adjacent one end thereof and inboard from a peripheral side edge **52** of the absorbent composite. Likewise, a pair of rear, side body panels **110** have inboard edges **184** that are secured to opposite side regions **186** of the absorbent composite, preferably on the bodyside thereof, adjacent the opposite end thereof and inboard from the peripheral side edge **152**. The body panels **108, 110** extend laterally outward from the absorbent composite and form ear portions **112, 114** having outboard edges **188, 190**. It should be understood that the absorbent composite could alternatively be secured to the garment side of the body panels.

Referring to FIGS. 1, 5, 9, 13, 17 and 21, fastening tabs **42** are attached and extend laterally from the outboard edge **28, 190** of the rear body panels from an attachment location **45**. It should be understood that the fastening tabs could be affixed to the front body panels or to both the front and rear body panels. For the purposes of illustration, the right side tab **42** is shown as being folded in during manufacture, while the left side tab **42** is shown as being extended outboard during use. The fastening tabs can be made of a hook and loop combination, such as a VELCRO® fastening system, or can have adhesive or other bonding agents applied to one surface thereof. As shown in FIG. 1, the tab **42** can include one or more attachment pads **43**. Alternatively, the fastening tabs can include buttons, snaps, ties or other known fastening devices. The tabs can be secured to the body panel with adhesive bonds, sonic bonds, thermal bonds, pinning, stitching or other known types of attachment.

When the absorbent garment is secured to the user, the fastening tabs **42** secured to the body panels **6, 110** on one end of the absorbent composite engage or are otherwise connected to the body panels **4, 108** on the opposite end of the garment. When secured in this way, openings **120** are formed on each side of the absorbent composite with the peripheral edge **52, 152** of the absorbent composite further defining the opening along the crotch region of the garment. When secured, the front and rear body panels **108, 110** form opposite side body panels.

It should be understood that the outboard edges **24, 28, 188, 190** of the front and rear body panels could be connected, for example by bonding or sewing, to create a seam of a pant garment. Alternatively, the front and rear body panels can be formed integrally, for example as a single side panel attached to opposite ends **116, 118** of the absorbent composite, or as one panel extending around the waist and hips of the user.

In particular aspects of the invention, either or all of the body panels may be composed of a wide range of materials with various basis weights and properties. For example, the body panel material may include knitted or other woven fabrics, nonwoven fabrics, polymer films, laminates, and the like, as well as combinations thereof. Preferably, the body panels are made of an elastic material, wherein the term "elastic" means capable of recovering the size and shape thereof after deformation, and/or exhibiting a retractive force.

In the various configurations of the invention, the basis weight of the body panel material can be at least a minimum of about 10 g/m$^2$. Alternatively, the basis weight can be at least about 20 g/m$^2$, and optionally, can be at least about 40 g/m$^2$ to provide improved benefits. In further aspects, the basis weight of the body panel material can be not more than a maximum of about 100 g/m$^2$. Alternatively, the basis weight can be not more than about 80 g/m$^2$, and optionally, can be not more than about 60 g/m$^2$ to provide improved performance.

In the differing configurations of the invention, the body panel material may be substantially permeable to air or substantially impermeable to air. The body panel material also may be substantially liquid-permeable or substantially liquid-impermeable. In particular arrangements, the body panel material may be substantially nonelastomeric. In other aspects, the body panels can include an elastomeric material which is elastomerically stretchable at least along the lateral article width. Examples of such elastomeric materials can include a neck-bonded-laminate (NBL), a stretch-bonded-laminate (SBL), a necked-thermal laminate, or the like, as well as combinations thereof. Such laminates can provide an improved combination of cloth-like feel and elastomeric stretchability. The body panels can be composed of materials that are elastic or elastomeric and exhibit biaxial stretch characteristics or MD/CD stretch characteristics, or that are extensible composites.

In desired configurations, the elastomeric body panel material can provide an elastomeric, stretch elongation which is at least about 20%, and desirably is at least about 50%. Alternatively, the stretch elongation can be at least about 75 %, and optionally, can be at least about 100% to provide improved performance. In other aspects, the elastomeric stretch elongation can be not more than about 400% and desirably can be not more than about 200%. Alternatively, the stretch elongation can be not more than about 300%, and optionally can be not more than about 250% to provide improved performance.

The percentage of elastomeric stretch or other elongation can be determined in accordance with the following formula:

$$100 * (L - L_0)/L_0;$$

where:

L = stretched length,
$L_0$ = initial length,

In addition, the amount of stretch elongation is determined under an applied tension force of 250 gram-force per inch of width measured perpendicular to the direction of the applied tension. Various configurations and materials of the body panels are further shown and described in U.S. Patent No. 6,132,410 directed to a "Disposable Garment Having Dryness Barriers With Expandable Attachment To An Absorbent".

The body panels also can be made of extensible materials, or combinations of elastomeric and extensible materials. It should be readily appreciated that each of the individual body panels may be composed of different materials, or of substantially the same material.

The term extensible means capable of being extended, and that it provides a selected elongation when subjected to an applied tensile force. The body panel also is preferably capable of providing a selected, sustained deformation when subjected to an applied tensile force and then allowed to relax for a selected time period beginning immediately after removal of the tensile force. Preferably the sustained deformation is substantially permanent deformation. The selected elongation and sustained deformation preferably occur at least along the lateral cross-direction of the garment, although it should be understood that it also could occur along the longitudinal direction, or both. Various extensible materials, and other acceptable materials that can be used for the body panels and the absorbent composite, which may include without limitations a retention portion, a topsheet and a backsheet, are described in U.S. Application S/N 09/249,434 filed February 12, 1999, entitled Expandable Cover Garment.

Referring again to FIGS. 1-4, the absorbent composite **50** has first and second longitudinally opposed end regions **56, 58** and first and second longitudinally opposed terminal end edges **60, 62**. The absorbent composite includes a substantially liquid permeable topsheet **64,** or liner, and a substantially liquid impermeable backsheet **68,** or barrier layer. A retention portion **70** is disposed or sandwiched between the topsheet and the backsheet, which are connected, at least along their peripheral edges. The topsheet and backsheet can be minimally attached, e.g. at the peripheral edges, or they can be attached across substantially the entire surface area thereof. The topsheet and backsheet can be joined for example with adhesive bonds, sonic bonds, thermal bonds, pinning, stitching or any other attachment techniques known in the art, as well as combinations thereof. For example, a uniform continuous layer of adhesive, a patterned layer of adhesive, a sprayed pattern of adhesive or any array of lines, swirls or spots of construction bonds may be used to join the topsheet and backsheet, or any of the other components described herein. Additional layers, including for example, a surge layer **72,** can also be incorporated into the absorbent composite. Preferably, the surge layer does not run the entire length of the absorbent composite and is shorter than the retention portion. In alternative configurations, the topsheet is indirectly joined to the backsheet by affixing the topsheet to intermediate layers, such as the surge layer or retention portion, which in turn is affixed to the backsheet.

The topsheet **64** presents a body-facing surface that is compliant, soft-feeling, and non-irritating to the wearer's skin. Further, the topsheet **64** can be less hydrophilic than retention portion **20,** and is sufficiently porous to be liquid permeable, permitting liquid to readily penetrate through its thickness to reach the retention portion. A suitable topsheet layer **64** may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The topsheet layer **64** is typically employed to help isolate the wearer's skin from liquids held in the retention portion.

Various woven and nonwoven fabrics can be used for topsheet **64.** For example, the topsheet may be composed of a meltblown or spunbonded web of the desired fibers, and may also be a bonded-carded-web. The various fabrics can be composed of natural fibers, synthetic fibers or combinations thereof.

For the purposes of the present description, the term "nonwoven web" means a web of fibrous material which is formed without the aid of a textile weaving or knitting process. The term "fabrics" is used to refer to all of the woven, knitted and nonwoven fibrous webs.

The topsheet fabrics may be composed of a substantially hydrophobic material, and the hydrophobic material may optionally be treated with a surfactant or otherwise process to impart a desired level of wettability and hydrophilicity. In a particular embodiment of the invention, topsheet 64 is a nonwoven, spunbond polypropylene fabric composed of about 2.8 - 3.2 denier fibers formed into a web having a basis weight of about 22 gsm and density of about 0.06 gm/cc. The fabric can be surface treated with an operative amount of surfactant, such as about 0.28 % Triton X-102 surfactant. The surfactant can be applied by any conventional means, such as spraying, printing, brush coating or the like.

In various embodiments, as described below, the topsheet can be made of extensible materials, as described with respect to the body panels and backsheet. For example, the topsheet can be prenecked for extensibility.

The backsheet **68** is preferably liquid impermeable, but may be liquid permeable, e.g., when a barrier layer is used with the retention portion. For example, in one embodiment, the backsheet can be made from a thin plastic film, or other flexible, substantially liquid-impermeable material. As used herein, the term "flexible" means a material that is compliant and which will readily conform to the general shape and contour of the body of the user. The backsheet prevents various bodily fluids and exudates from wetting or otherwise contaminating various bedding or outer garments worn by the user over the absorbent garment. In particular, the backsheet can include a film, such as a polyethylene film, having a thickness of from about 0.012 mm to about 0.051 mm.

In other alternative constructions, the backsheet can comprise a woven or nonwoven fibrous web layer, which is treated or constructed, partially or wholly, to impart the desired levels of liquid impermeability to selected regions that are adjacent to or proximate the absorbent retention portion. For example, the backsheet may include a gas-permeable, nonwoven fabric layer laminated to a polymer film layer which may or may not be gas-permeable. Other examples of fibrous, cloth-like backsheet materials can comprise a stretch thinned or stretch thermal laminate material composed of a 0.6 mil (0.015 mm) thick polypropylene cast film and a 0.7 ounce per square yard (23.8 gsm) polypropylene spunbond material (2 denier fibers). A material of this type has been employed to form the outercover of a HUGGIES® Ultratrim Disposable Diaper, which has been commercially available from Kimberly-Clark Corporation. The backsheet **68** typically provides the outercover of the article. Optionally, however, the article may include a separate outercover component member which is additional to the backsheet. The outercover can be joined, for example, to one or more of the aborbent composite and/or body panels.

The backsheet may include a micro-porous, "breathable" material which permits gases, such as water vapor, to escape from the absorbent garment while substantially preventing liquid exudates from passing through the backsheet. For example, the breathable backsheet may be composed of a microporous polymer film or a nonwoven fabric which has been coated or otherwise modified to impart a desired level of liquid impermeability. For example, a suitable microporous film can be a PMP-1 material, which is available from Mitsui Toatsu Chemicals, Inc., a company having offices in Tokyo, Japan; or an XKO-8044 polyolefin film available from 3M Company of Minneapolis, Minnesota. The backsheet may also be embossed or otherwise provided with a pattern or matte finish to exhibit a more aesthetically pleasing appearance.

In various configurations of the invention, where a component, such as the backsheet is configured to be permeable to gas while having a resistance and limited permeability to aqueous liquid, the liquid resistant component can have a construction which is capable of supporting a selected hydrohead of water substantially without leakage therethrough. A suitable technique for determining the resistance of a material to liquid penetration is Federal Test Method Standard FTMS 191 Method 5514, 1978, or an equivalent thereof.

In one preferred embodiment, the backsheet is sufficiently impermeable to liquid and semi-liquid materials to substantially prevent the undesired leakage of waste materials, defined as exudates, including for example urine and feces. For example, the backsheet member can desirably support a hydrohead of at least about 45 centimeters (cm) substantially without leakage. The backsheet member can alternatively support a hydrohead of at least about 55 cm, and optionally, can support a hydrohead of at least about 60 cm, or more, to provide improved benefits.

The backsheet is preferably extensible, as that term is defined above with respect to the body panels. In one preferred embodiment, the backsheet is capable of providing an elongation of at least about 1 cm when subjected to a tensile force of 11.8 g/cm, and further provides a substantially permanent deformation of at least about 20% when subjected to a tensile force of 19.70 g/cm and is then allowed to relax under a zero applied stress for a period of 1 minute.

For example, the extensible backsheet can be composed of a necked fiber, a creped fiber, a micro-pleated fiber, polymer films or the like, as well as combinations thereof. The fabrics may be woven or nonwoven materials, such as spunbond fabrics. One example of a suitable extensible material is a 60% necked, polypropylene spunbond having a basis weight of about 1.2 osy.

The backsheet also can be expandable, for example when it has one or more folds, e.g., one or more z-folds (not shown), or can be both extensible and expandable. The term expandable as used herein means to enlarge or to increase the extent or area, lateral and/or longitudinal, thereof, e.g., by unfolding one or more folds.

The retention portion **70** is preferably made of an absorbent material, which tends to swell or expand as it absorbs liquid excreted or exuded by the user. For example, the absorbent material can be made of airformed, airlaid and/or wetlaid composites of fibers and high absorbency materials, referred to as superabsorbents. Superabsorbents typically are made of polyacrylic acids, such as FAVOR 880 available from Stockhausen, Inc. of Greensboro, North Carolina. The fibers can be fluff pulp materials, such as Alliance CR-1654, or any combination of crosslinked pulps, hardwood, softwood, and synthetic fibers. Airlaid and wetlaid structures typically include binding agents, which are used to stabilize the structure. In addition, various foams, absorbent films, and superabsorbent fabrics can be used as an absorbent material.

In one preferred embodiment, an absorbent material is made of fibrous absorbent materials with a relatively high internal integrity, including for example one made with thermoplastic binder fibers in airlaid absorbents, e.g., pulp, bicomponent binding fibers, and superabsorbents, which have higher densities in the folded regions. The higher density and resulting smaller capillary size in these regions promotes better wicking of the liquid. Better wicking, in turn, promotes higher utilization of the absorbent material and tends to result in more uniform swelling throughout the absorbent material as it absorbs the liquid.

Various types of wettable, hydrophilic fibrous material can be used to form the component parts of absorbent, and particularly the retention portion **70**. Examples of suitable fibers include naturally occurring organic fibers composed of intrinsically wettable material, such as cellulosic fibers; synthetic fibers composed of cellulose or cellulose derivatives, such as rayon fibers; inorganic fibers composed of an inherently wettable material, such as glass fibers; synthetic fibers made from inherently wettable thermoplastic polymers, such as particular polyester or polyamide fibers; and synthetic fibers composed of a nonwettable thermoplastic polymer, such as polypropylene fibers, which have been hydrophilized by appropriate means. The fibers may be hydrophilized, for example, by treatment with silica, treatment with a material which has a suitable hydrophilic moiety and is not readily removable from the fiber, or by sheathing the nonwettable; hydrophobic fiber with a hydrophilic polymer during or after the formation of the fiber. For the purposes of the present invention, it is contemplated that selected blends of the various types of fibers mentioned above may also be employed.

As used herein, the term "hydrophilic" describes fibers or the surfaces of fibers which are wetted by the aqueous liquids in contact with the fibers.

The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System, or a substantially equivalent system. When measured with such system, fibers having contact angles less than 90° are designated "wettable", while fibers having contact angles greater than 90° are designated "nonwettable".

In particular arrangements, the retention portion of the absorbent may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness (z-direction) of the absorbent structure, with lower concentrations toward the bodyside of the absorbent composite and relatively higher concentrations toward the outside of the absorbent structure. Suitable z-gradient configurations are described in U.S.P. 4,699,823 issued October 13, 1987 to Kellenberger et al.

Alternatively, the concentrations of superabsorbent particles may be arranged in a non-step-wise gradient, through a substantial portion of the thickness (z-direction) of the absorbent structure, with higher concentrations toward the bodyside of the absorbent composite and relatively lower concentrations toward the outside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

The high-absorbency material may comprise absorbent gelling materials, such as superabsorbents. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bods, ionic complexes and associations, hydrophilic associations, such as hydrogen bonding, and hydrophobic associations or Van der Walls forces.

Examples of synthetic absorbent gelling material polymers include the alkali metal and ammonium salts of poly(acrylic acid) and poly (methacrylic acid), poly (acrylamides), poly(vinyl ethers), maleic anhydride copolymers with vinyl ethers and alpha-olefins, poly(vinyl pyrrolidone), poly(vinylmorpholinone), poly(vingyl alcohol), and mixtures and copolymers thereof. Further polymers suitable for use in the absorbent composite include natural and modified natural polymers, such as hydrolyzed acrylonitrile-grafted starch, acrylic acid grafted starch, methyl cellulose, chitosan, carboxymethyl cellulose, hydroxypropyl cellulose, and the natural gums, such as alginates, xanthan gum, locust bean gum and the like. Mixtures of natural and wholly or partially synthetic absorbent polymers can also be useful in the present invention. Other suitable absorbent gelling materials are disclosed by Assarsson et al. in U.S. Patent No. 3.901,236 issued August 26, 1975. Processes for preparing synthetic absorbent gelling polymers are disclosed in U.S. Patent No. 4,076,663 issued February 28, 1978 to Masuda et al. and U.S. Patent No. 4,286,082 issued August 25, 1981 to Tsubakimoto et al.

Synthetic absorbent gelling materials typically are xerogels which form hydrogels when wetted. The term "hydrogel", however, has commonly been used to also refer to both the wetted and unwetted forms of the material.

As mentioned previously, the high-absorbency material used in the absorbent is generally in the form of discrete particles. The particles can be of any desired shape, for example, spiral or semi-spiral, cubic, rod-like, polyhedral, etc. Shapes having a large greatest dimension/smallest dimension ration, like needles, flakes and fibers, are also contemplated for use herein. Conglomerates of particles of absorbent gelling material may also be used in the absorbent. Desired for use are particles having an average size of from about 20 microns to about 1 millimeter. "Particle size" as used herein means the hydrophilic fibers and high-absorbency particles can be configured to form an average composite basis weight which is within the range of about 50 -1500 gsm. In certain aspects of the invention, the average composite basis weight is within the range of about 200 - 1200 gsm, and alternatively is within the range of about 200 -1200 gsm, and alternatively is within the range of about 500 -800 gsm to provide desired performance. Furthermore, the proportion of high absorbency particles can range from about 0 to about 100% and the proportion of fibrous material from about 0 to about 100%. Additionally, high absorbency fibers can be used such as Oasis Type 121 and Type 122 superabsorbent fibers available from Technical Absorbent Ltd., Grimsby, Lincolnshire, United Kingdom.

The retention portion **70** has laterally opposed side edges **74** and preferably is made of a single layer of material. The retention portion preferably has an hour-glass shape with enlarged end regions. Alternatively, the retention portion can include a folded or multilayered configuration. The retention portion preferably has a length substantially equal to, or slightly shorter than, the length of the absorbent composite. The retention portion can include one or more barrier layers attached to the absorbent material. In one embodiment, an upper and lower tissue substrate **88, 90** also can be disposed adjacent the retention portion, or alternatively the tissue can completely envelope the retention position.

Referring to FIG. 1, the opposite garment side of the end regions **56, 58** of the absorbent composite, and in particular, the outer, garment side surface of the backsheet **68,** are secured to the bodyside surface of the longitudinally opposed crotch ends of the first and second body panels **4, 6**. Preferably, the end regions **56, 58** are connected to the bodyside surface of the body panels along three longitudinally extending, parallel, spaced locations **76, 78**. It should be understood that the absorbent composite can be secured using any of the methods of attachment described above. Moreover, it should be understood that the three locations are meant to be illustrative, and that the lateral width (W) of the absorbent composite defined between the opposite outboard edges of attachment can be secured to the body panels with any configuration of attachment lines, swirls, patterns, spots, etc., or can be a full and continuous attachment therebetween. The outboard attachment locations **76,** or the outboard edge of the area of attachment, are spaced inboard from the side edge **52** of the absorbent composite, slightly outboard of the side edges **74** of the retention portion. The retention portion **70** has a lesser lateral width than the overall absorbent composite.

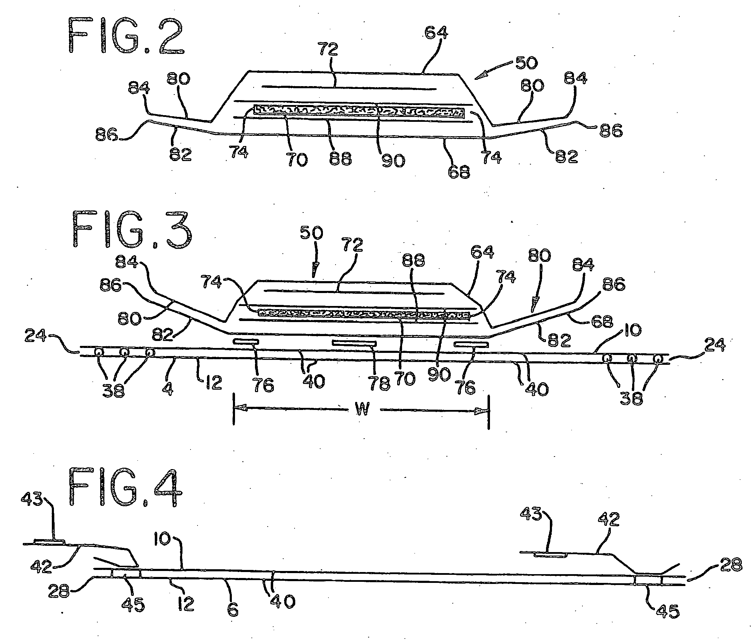

In particular, in the embodiment shown in FIGS. 1-4, the backsheet and topsheet have laterally extending side margins **82, 80** that extend laterally outboard from the side edge **74** of the retention portion and from the location **76** of attachment to the body panels. It should be understood that the term "location" means any region, dot, position or side, and is not limited to the longitudinally extending lines shown in the Figures. For example, the location could comprise a series of longitudinally extending dots or points. Conversely, the location could comprise a continuous region or area attachment extending across the lateral width (W) of the absorbent composite between the outboard edges of the attached region of the absorbent composite. The term "attached region" means the region extending between the opposite outboard edge of the attachment locations, and is shown, for example, as having a width W in FIG. 3. The side margins **80, 82** are preferably formed along the entire longitudinal extent of the portion of the absorbent composite that overlaps the body panels, and also extend along the crotch and the absorbent composite between the body panels. However, it should be understood that the side margins may extend along only a portion of the overlapping absorbent composite. The side margins **80, 82** are not attached to the body panels 4, 6 and terminate in free edges **84, 86**.

It should be understood that in an alternative embodiment, one or more other layers including for example the surge layer or tissue layers, also can have opposite side margins that extend outboard from the locations **76** of attachment to the body panels and are sandwiched between the side margins **80, 82**.

The side margins preferably have a lateral width greater than about 3 mm and less than about 75 mm. More preferably, the side margins each have a width of between about 15 mm and about 45 mm, and more preferably a width of between about 25 mm and about 35mm. Preferably, the side margins have a combined lateral width that is at least 3% and not more than about 75 % of the total lateral width of the absorbent composite, and more preferably between about 15% and 50% of the total lateral width, and even more preferably between about 25% and 35%. The body panels extend beyond the lateral and longitudinal edges of the side margins of the absorbent composite.

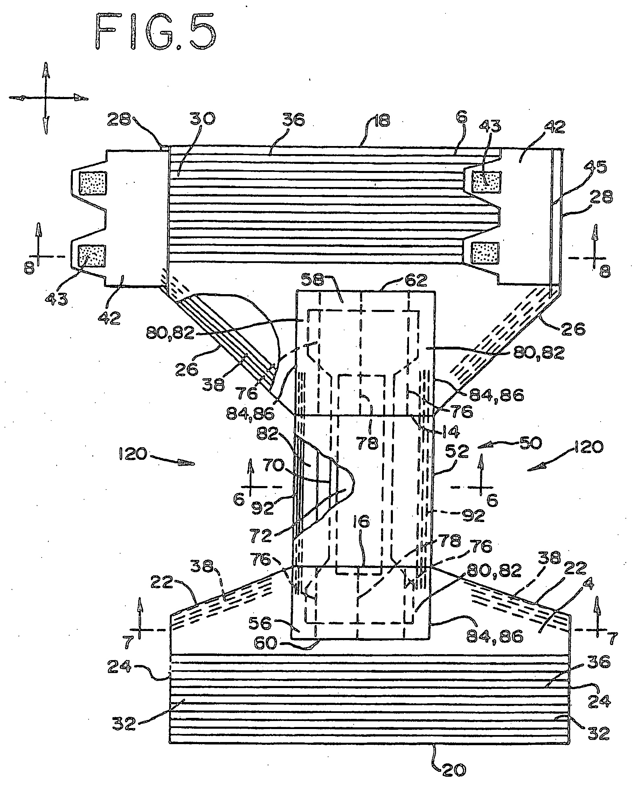

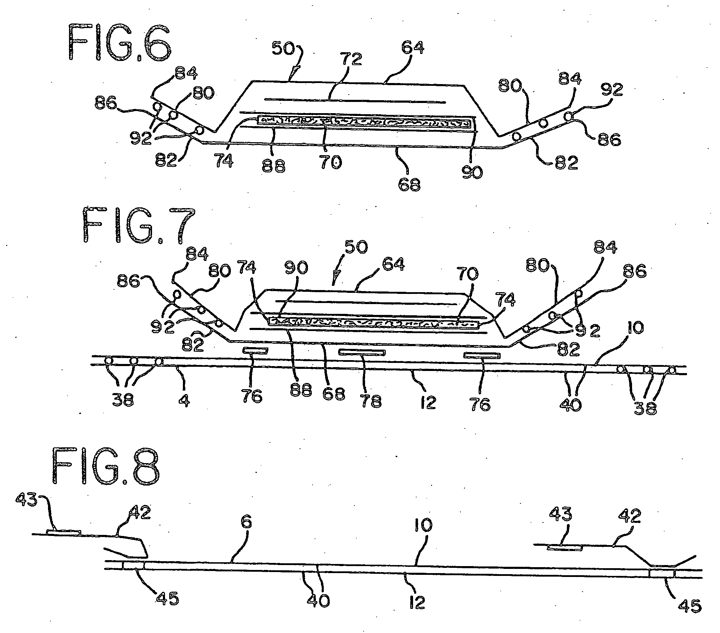

In a second embodiment shown in FIG. 5-8, one or more elastic elements **92,** shown as there, are secured in the side margins **84, 86** between the topsheet and backsheet, and extend longitudinally along a portion of the side margins on each side of the absorbent composite. Preferably, the elastic elements extend along the side margins of the absorbent composite between the body panels and overlap a portion of each body panel **4, 6.** The length of the elastic elements is preferably between about 5% and 100% of the length of the absorbent composite. The function of the elastic elements in the side margins of the absorbent composite are to shorten the length of the side margin which pulls the side margins inwardly to form a three-dimensional profile. The elastic elements can be positioned at various laterally spaced positions, depending on the amount of shortening and upward lift desired. The elastic elements can be made of ribbon, films, sprays of elastic, or other elastic configurations know in the art. It should be understood that elastic elements can be incorporated into any of the other side margin configurations described herein.

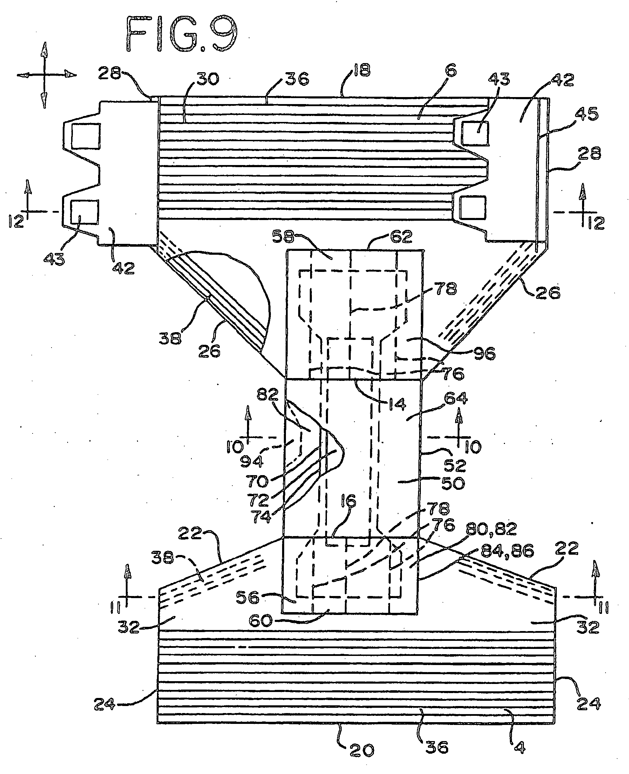

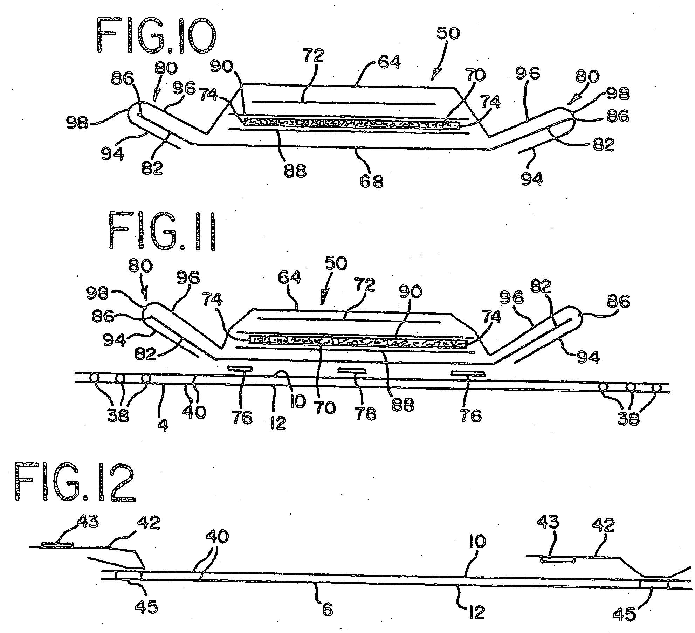

In a third embodiment shown in FIGS. 9-12, the topsheet side margin **80** includes a first portion **94** folded over a second portion **96,** with a side margin portion **82** of the backsheet disposed between the first and second portions **94, 96** of the topsheet. The folded first and second portions **94, 96** form a folded edge **98** that defines the free edge of the side margin of the absorbent composite. The function of the folded topsheet is to provide a clothlike feel to the portion **82** of the backsheet that forms the side margin. Preferably, the topsheet covers at least the free edge **86** of the backsheet, which provides comfort to the user with minimal cost.

In yet another arrangement shown in FIGS. 25-28, the body panel **300** is continuous from the rear of the garment through the crotch region to the front of the garment. In such an arrangement, the absorbent composite can be attached to the front **302,** rear **304** and crotch **306** portions of the body panel. In essence, a crotch body panel extends between and connects a front and rear body panel. The body panel **300** is preferably made of continuous sheets or layers that form the three regions, although it should be understood that separate pieces can be joined, e.g., by bonding, stitching etc., to form the full length body panel.

In one embodiment, the two locations **172** of attachment would extend the length of the absorbent composite. In one arrangement, shown in FIG. 26, the body panel width in the crotch region **306,** defined by outboard edges **310,** extends between the outboard attachment locations, or is preferably substantially the same width as the width "W" of the attached region, and more preferably is substantially the same width as the retention portion. In an alternative arrangement, the body panel extends outboard from the attachment locations, but remains unattached to the side margins of the absorbent composite. In the arrangements of Figures 25-28, the absorbent composite extends substantially the entire length of the garment and such arrangements are therefore not in accordance with the invention. In alternative embodiments in accordance with the invention, it should be understood that the absorbent composite could have a length less than the entire length of the garment, as shown for example in FIG. 1.

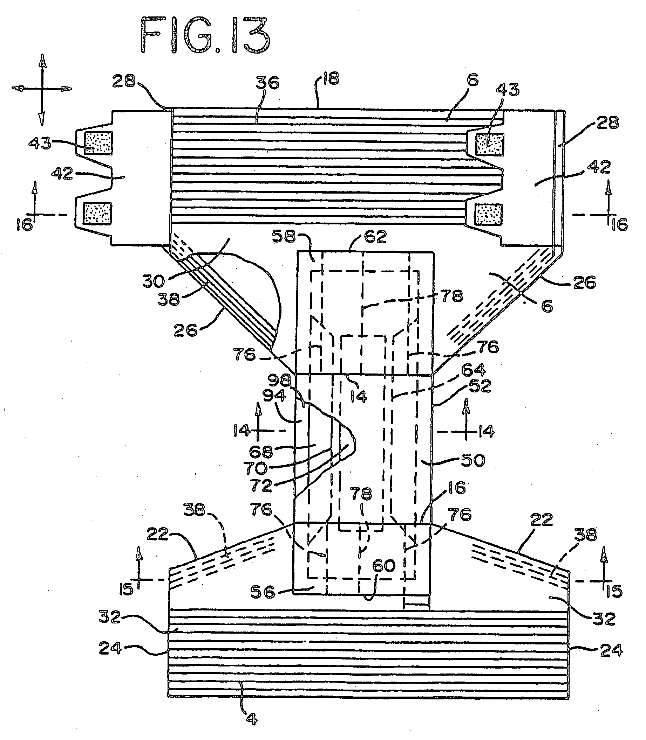

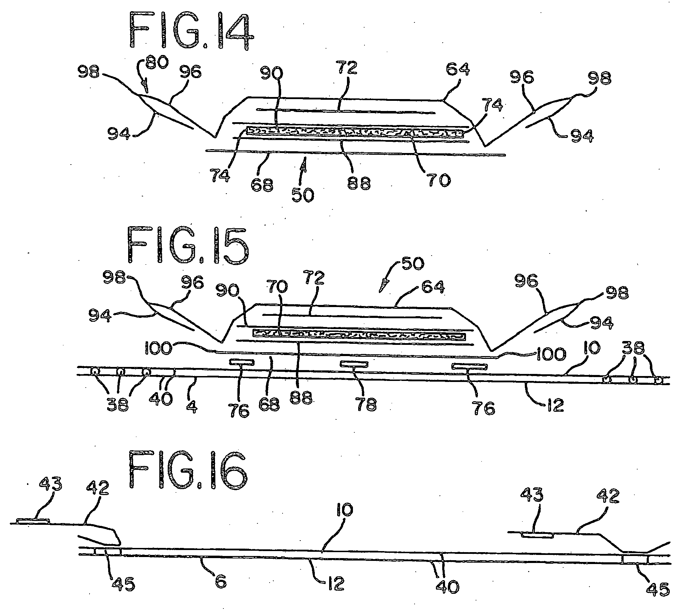

In another embodiment, shown in FIGS. 13-16, the side margin is formed only by the topsheet, with the backsheet having side edges terminating proximate the attachment locations. Preferably, the topsheet has a folded first and second portion **94, 96** forming a folded edge **98** that defines of the side margin. Conversely, it should be understood that the side margin can be formed only by the backsheet, which can be monolayer, or which can have various folds, including for example a folded first and second portion. It should be understood that the first portion can be folded under the second portion, as shown in the Figures, or folded over the second portion. It also should be understood that the side margin could have additional folds, formed for example by a third portion interfolded with the first and second portions. Preferably, each side margin or the side margins in combination have a width or range of widths as described above.

In another embodiment, shown in FIGS. 17-20, the side margin **80** is tapered from a first width D adjacent a first position **102** at the terminal edge of the body panel to a second width D2 adjacent a second position **104** at the end of the end edge of the absorbent composite. In one embodiment, the second width is zero. Preferably, D ranges from not less than about 5% to not greater than about 50% of the total lateral width of the absorbent composite. Preferably, D2 ranges from about 0 to not greater than about 50% of the total lateral width of the absorbent composite. The gradient attachment location 106 allows the marginal side edges of the absorbent composite to fold inwardly in the crotch region during use, while the attachment at the end regions provides the area of coverage of the absorbent composite in the front and back panels without affecting the fit of the garment.

In another arrangement, shown in FIGS. 21-24, which is not in accordance with the invention, the absorbent composite **50** extends substantially the entire length of and defines the length of the absorbent composite and preferably the absorbent garment. Each of the bodyside surfaces of the inboard terminal edges **180, 184** of the front and back side panels **108, 110** are secured to the absorbent composite along a location **172** spaced laterally inboard from the side edge **52** of the absorbent composite so as to form opposite side margins **80, 82.** As such, the side margins extend the entire longitudinal length of the body panels and the garment. It should be understood that although the side margins **80, 82** are shown as being formed from the backsheet and topsheet, without a folded edge, any of the above-described side margin configurations could be used, including a folded topsheet configuration. Indeed, it should be understood that any of the aforementioned configurations of the side margins, whether formed from one or both of the backsheet and topsheet, with or without folded portions and with or without elastic elements, can be incorporated into an absorbent composite attached to any of the aforementioned body panel configurations, or their equivalents, including without limitation, a continuous one-piece body panel (whether made from one or more layers or plies), a two-piece body panel, or a four piece body panel.

Although the present invention has been described with reference to preferred embodiments, those skilled in the art will recognize that changes may be made in form and detail without departing from the scope of the invention. As such, it is intended that the foregoing detailed description be regarded as illustrative rather than limiting and that it is the appended claims, which are intended to define the scope of the invention.

**Claims**

1. An absorbent garment (2) comprising:

a body panel (4) having a bodyside surface (10) and a terminal waist edge (18); and
an absorbent composite (50) having a laterally extending width and a longitudinally extending length, a garment-side surface, first and second longitudinally opposed end regions (56, 58), and first and second longitudinally opposed terminal end edges (60, 62), the absorbent composite comprising a backsheet (68), a topsheet (64) and a retention portion (70) disposed between said backsheet and said topsheet, wherein the retention portion (70) has opposite lateral side edges (74), and wherein said garment-side surface of an end region of said absorbent composite is connected to said bodyside surface of said body panel along at least a longitudinally extending location (76), **characterised in that** said absorbent composite (50) has a length less than the entire length of the garment, and that said location of attachment (76), or the outboard edge thereof, is spaced inboard from a side edge (52) of the absorbent composite, and outboard of the side edge (74) of the retention portion (70);

said absorbent composite (50) comprising a longitudinally extending side margin (82) extending laterally outboard from the side edge of said retention portion and from said location of attachment (76) to said body panel, wherein said side margin is not attached to the body panel and terminates in a free edge, the side margin extending along at least a portion of the longitudinal extent of the absorbent composite (50) that overlaps said body panel (4), wherein said body panel comprises a first body panel (4) connected to said first end region (56) and a second body panel (6) connected to said second end region (58), wherein said first and second body panels are longitudinally spaced apart, and wherein said absorbent composite (50) is connected to said first body panel (4) along at least one longitudinally extending first location, and wherein said absorbent composite is connected to said second body panel (6) along at least one longitudinally extending second location, and wherein said side margin extends laterally outboard from each of said first and second locations.

2. The garment of claim 1 wherein the body panel (4) extends beyond the lateral and longitudinal edges of said side margin.

3. The garment of any preceding claim wherein the side margin (82) extends along the entire longitudinal extent of the portion of the absorbent composite (50) that overlaps the body panel.

4. The garment of claim 1 wherein said first body panel (4) comprises a first pair of side body panels and wherein said second body panel (6) comprises a second pair of side body panels, wherein each of said side body panels comprises an outboard terminal edge and an inboard terminal edge, wherein said at least one first location (76) comprises a pair of first locations and wherein said at least one second location (76) comprises a pair of second locations, and wherein said inboard terminal edges of each of said first pair of side body panels is attached to said absorbent composite at one of said pairs of said first locations respectively and wherein said inboard terminal edge of each of said second pair of side body panels is attached to said absorbent composite at one of said pairs of said second locations, and wherein said absorbent composite (50) comprises opposite side margins extending laterally outboard from each of said first and second locations.

5. The garment of any of claims 1 to 3 wherein said absorbent composite (50) comprises opposite side margins extending laterally outboard on each side of said absorbent composite (50) and terminating in opposite free edges.

6. The garment of any preceding claim wherein said side margin is formed from said backsheet (68).

7. The garment of any of claims 1 to 5 wherein said side margin is formed from said topsheet (64).

8. The garment of claim 7 wherein side margin comprises a first portion of said topsheet (64) folded over a second portion of said topsheet, wherein said folded first and second portions form a folded edge defining said free edge of said side margin.

9. The garment of claim 8 wherein said side margin further comprises a portion of said backsheet (68) disposed between said first and second portions of said topsheet.

10. The garment of any of claims 1 to 5 wherein said side margin is formed from said topsheet (64) and said backsheet (68).

**11.** The garment of any preceding claim wherein said side margin(s) comprise a longitudinally extending elastic element.

**12.** The garment of any preceding claim wherein the or each side margin (80) has a width greater than 3 mm and less than 75 mm, preferably between about 15 mm and about 45 mm, and most preferably between about 25 mm and about 35 mm.

**13.** The garment of any preceding claim wherein the absorbent composite comprises opposite side margins extending laterally outboard on each side of said absorbent composite (50) and terminating in opposite free edges, and the side margins have a combined width that is at least 3% and not more than about 75% of the total lateral width of the absorbent composite, more preferably between about 15 % and 50 % of the total lateral width of the absorbent composite, and more preferably between about 25 % and 35 % of the total lateral width of the absorbent composite.

**Patentansprüche**

**1.** Absorbierendes Kleidungsstück (2), das aufweist:

eine Körperbahn (4), die eine körperseitige Fläche (10) und eine Abschluss-Taillenkante (18) besitzt; und
ein absorbierendes Komposit (50), das eine sich seitlich erstreckende Breite und eine sich längs erstreckende Länge, eine Oberfläche auf der Seite des Kleidungsstücks, einen ersten und einen zweiten längs gegenüberliegenden Endbereich (56, 58) und eine erste und eine zweite längs gegenüberliegende Abschlussendkante (60, 62) besitzt, wobei das absorbierende Komposit eine Unterlageschicht (68), eine Oberseitenschicht (64) und einen Rückhalteabschnitt (70), der zwischen der Unterlageschicht und der Oberseitenschicht angeordnet ist, aufweist, wobei der Rückhalteabschnitt (70) gegenüberliegende seitliche Seitenkanten (74) besitzt, und wobei die Oberfläche auf der Seite des Kleidungsstücks eines Endbereichs des absorbierenden Komposits mit der körperseitigen Fläche der Körperbahn entlang mindestens einer sich längs erstreckenden Stelle (76) verbunden ist, **dadurch gekennzeichnet, dass** das absorbierende Komposit (50) eine Länge geringer als die gesamte Länge des Kleidungsstücks besitzt und dass die Stelle der Befestigung (76), oder die außen liegende Kante davon, nach innen von einer Seitenkante (52) des absorbierenden Komposits, und nach außen von der Seitenkante (74) des Rückhalteabschnitts (70), beabstandet ist;

wobei das absorbierende Komposit (50) einen sich längs erstreckenden Seitenrand (82) aufweist, der sich seitlich nach außen von der Seitenkante des Rückhalteabschnitts und von der Stelle der Befestigung (76) zu der Körperbahn hin erstreckt, wobei der Seitenrand nicht an der Körperbahn befestigt ist und in einer freien Kante endet, wobei sich der Seitenrand entlang mindestens eines Abschnitts der Längserstreckung des absorbierenden Komposits (50), der die Körperbahn (4) überlappt, erstreckt, wobei die Körperbahn eine erste Körperbahn (4), die mit dem ersten Endbereich (56) verbunden ist, und eine zweite Körperbahn (6), die mit dem zweiten Endbereich (58) verbunden ist, aufweist, wobei die erste und die zweite Körperbahn längs voneinander beabstandet sind und wobei das absorbierende Komposit (50) mit der ersten Körperbahn (4) entlang mindestens einer sich längs erstreckenden ersten Stelle verbunden ist und wobei das absorbierende Komposit mit der zweiten Körperbahn (6) entlang mindestens einer sich längs erstreckenden zweiten Stelle verbunden ist und wobei sich der Seitenrand seitlich außen von sowohl der ersten als auch der zweiten Stelle erstreckt..

**2.** Kleidungsstück nach Anspruch 1, wobei sich die Körperbahn (4) über die seitliche Kante und die Längskante des Seitenrands hinaus erstreckt.

**3.** Kleidungsstück nach einem vorhergehenden Anspruch, wobei sich der Seitenrand (82) entlang der gesamten Längserstreckung des Abschnitts des absorbierenden Komposits (50), der die Körperbahn überlappt, erstreckt.

**4.** Kleidungsstück nach Anspruch 1, wobei die erste Körperbahn (4) ein erstes Paar von Seitenkörperbahnen aufweist und wobei die zweite Körperbahn (6) ein zweites Paar von Seitenkörperbahnen aufweist, wobei jede der Seitenkörperbahnen eine außen liegende Endkante und eine innen liegende Endkante aufweist, wobei die mindestens eine erste Stelle (76) ein Paar erster Stellen aufweist und wobei die mindestens eine zweite Stelle (76) ein Paar zweiter Stellen aufweist, und wobei die innen liegenden Endkanten jedes des ersten Paars der Seitenkörperbahnen an dem absorbierenden Komposit jeweils an einem der Paare der ersten Stellen verbunden ist, und wobei die innen liegende Endkante jedes des zweiten Paars der Seitenkörperbahnen an dem absorbierenden Komposit an einem der Paare der zweiten Stellen befestigt ist, und wobei das absorbierende Komposit (50) gegenüberliegende Seitenränder aufweist, die sich seitlich außen von jeder der ersten und der zweiten Stelle erstrecken.

**5.** Kleidungsstück nach einem der Ansprüche 1 bis 3, wobei das absorbierende Komposit (50) gegenüberliegende Seitenränder aufweist, die sich seitlich außen an jeder Seite des absorbierenden Komposits (50) erstrekken und in gegenüberliegenden freien Kanten enden.

**6.** Kleidungsstück nach einem vorhergehenden Anspruch, wobei der Seitenrand aus der Unterlageschicht (68) gebildet ist.

**7.** Kleidungsstück nach einem der Ansprüche 1 bis 5, wobei der Seitenrand aus der Oberseitenschicht (64) gebildet ist.

**8.** Kleidungsstück nach Anspruch 7, wobei der Seitenrand einen ersten Bereich der Oberseitenschicht (64), der über einen zweiten Bereich der Oberseitenschicht gefaltet ist, aufweist, wobei der gefaltete erste und der zweite Bereich eine gefaltete Kante bilden, die die freie Kante des Seitenrands definiert.

**9.** Kleidungsstück nach Anspruch 8, wobei der Seitenrand weiterhin einen Bereich der Unterlageschicht (68), der zwischen dem ersten und dem zweiten Bereich der Oberseitenschicht angeordnet ist, aufweist.

**10.** Kleidungsstück nach einem der Ansprüche 1 bis 5, wobei der Seitenrand aus der Oberseitenschicht (64) und der Unterlageschicht (68) gebildet ist.

**11.** Kleidungsstück nach einem vorhergehenden Anspruch, wobei der Seitenrand (die Seitenränder) ein sich längs erstreckendes elastisches Element aufweist (aufweisen).

**12.** Kleidungsstück nach einem vorhergehenden Anspruch, wobei der oder jeder Seitenrand (80) eine Breite größer als 3 mm und geringer als 75 mm, vorzugsweise zwischen ungefähr 15 mm und ungefähr 45 mm, und am bevorzugtesten zwischen ungefähr 25 mm und ungefähr 35 mm, besitzt.

**13.** Kleidungsstück nach einem vorhergehenden Anspruch, wobei das absorbierende Komposit gegenüberliegende Seitenränder aufweist, die sich seitlich außen an jeder Seite des absorbierenden Komposits (50) erstrecken und in gegenüberliegenden freien Kanten enden, und die Seitenränder eine kombinierte Breite haben, die
mindestens 3 % und nicht mehr als ungefähr 75 % der gesamten seitlichen Breite des absorbierenden Komposits, bevorzugter zwischen ungefähr 15 % und 50 % der gesamten seitlichen Breite des absorbierenden Komposits und am bevorzugtesten zwischen ungefähr 25 % und 35 % der gesamten seitlichen Breite des absorbierenden Komposits beträgt.

**Revendications**

**1.** Vêtement absorbant (2) comprenant :

un panneau corporel (4) ayant une surface côté corporel (10) et un bord de ceinture terminal (18) ; et
un composite absorbant (50) ayant une largeur s'étendant transversalement et une longueur s'étendant longitudinalement, une surface côté vêtement, des première et seconde régions d'extrémité (56, 58) longitudinalement opposées, et des premier et second bords d'extrémité terminaux (60, 62) longitudinalement opposés, le composite absorbant comprenant une feuille-dossier (68), une feuille supérieure (64) et une portion de confinement (70) disposée entre ladite feuille-dossier et ladite feuille supérieure, la portion de confinement (70) ayant des bords latéraux (74) transversalement opposés, ladite surface côté vêtement d'une région d'extrémité dudit composite absorbant étant connectée à ladite surface côté corporel dudit panneau corporel le long d'au moins un emplacement (76) s'étendant longitudinalement, **caractérisé en ce que** ledit composite absorbant (50) a une longueur inférieure à la longueur totale du vêtement et **en ce que** ledit emplacement (76) de fixation, ou son bord côté extérieur, est espacé et en deçà d'un bord latéral (52) du composite absorbant et espacé et au-delà du bord latéral (74) de la portion de confinement (70)
ledit composite absorbant (50) comprenant une marge latérale (82), s'étendant longitudinalement, et qui s'étend, transversalement, au-delà du bord latéral de ladite portion de confinement et dudit emplacement de fixation (76) audit panneau corporel, ladite marge latérale n'étant pas fixée au panneau corporel et se terminant par un bord libre, la marge latérale s'étendant le long d'au moins une portion de l'étendue longitudinale du composite absorbant (50) qui chevauche ledit panneau corporel (4), ledit panneau corporel comprenant un premier panneau corporel (4) connecté à ladite première région d'extrémité (56) et un second panneau corporel (6) connecté à ladite seconde région d'extrémité (58), lesdits premier et second panneaux corporels étant espacés longitudinalement l'un de l'autre et ledit composite absorbant (50) étant connecté audit premier panneau corporel (4) le long d'au moins un premier emplacement s'étendant longitudinalement et ledit composite absorbant étant connecté audit second panneau corporel (6) le long d'au moins un second emplacement s'étendant longitudinalement, ladite marge latérale s'étendant transversalement vers l'extérieur depuis chacun desdits premier et second emplacements.

2. Vêtement selon la revendication 1 dans lequel le panneau corporel (4) s'étend au-delà des bords transversaux et longitudinaux de ladite marge latérale.

3. Vêtement selon l'une quelconque des revendications précédentes, dans lequel la marge latérale (82) s'étend le long de toute l'étendue longitudinale de la portion du composite absorbant (50) qui chevauche le panneau corporel.

4. Vêtement selon la revendication 1 dans lequel ledit premier panneau corporel (4) comprend une première paire de panneaux corporels latéraux et dans lequel ledit second panneau corporel (6) comprend une seconde paire de panneaux corporels latéraux, chacun desdits panneaux corporels latéraux comprenant un bord terminal, vers l'extérieur, et un bord terminal, vers l'intérieur, ledit au moins un emplacement (76) comprenant une paire de premiers emplacements et ledit au moins un second emplacement (76) comprenant une paire de seconds emplacements, lesdits bords terminaux, vers l'intérieur, de chacune desdites premières paires de panneaux corporels latéraux étant respectivement fixés audit composite absorbant au niveau de l'une desdites paires de premiers emplacements, et ledit bord terminal, vers l'intérieur, de chacune desdites secondes paires de panneaux corporels latéraux étant fixé audit composite absorbant au niveau de l'une desdites paires desdits seconds emplacements, ledit composite absorbant (50) comprenant des marges latérales opposées s'étendant transversalement vers l'extérieur à partir de chacun desdits premier et second emplacements.

5. Vêtement selon l'une quelconque des revendications 1 à 3, dans lequel ledit composite absorbant (50) comprend des marges latérales opposées s'étendant transversalement au-delà de chaque côté dudit composite absorbant (50) et se terminant en bords libres opposés.

6. vêtement selon l'une quelconque des revendications précédentes, dans lequel ladite marge latérale est formée à partir de ladite feuille-dossier (68).

7. Vêtement selon l'une quelconque des revendications 1 à 5, dans lequel ladite marge latérale est formée à partir de ladite feuille supérieure (64).

8. Vêtement selon la revendication 7, dans lequel la marge latérale comprend une première portion de ladite feuille supérieure (64) repliée sur une seconde portion de ladite feuille supérieure, lesdites première et secondes portions pliées formant un bord plié définissant ledit bord libre de ladite marge latérale.

9. Vêtement selon la revendication 8, dans lequel ladite marge latérale comprend en outre une portion de ladite feuille-dossier (68) disposée entre lesdites première et seconde portions de ladite feuille supérieure.

10. Vêtement selon l'une quelconque des revendications 1 à 5, dans lequel ladite marge latérale est formée à partir de ladite feuille supérieure (64) et de ladite feuille-dossier (68).

11. vêtement selon l'une quelconque des revendications précédentes, dans lequel ladite ou lesdites marge (s) latérale(s) comprend(nent) un élément élastique s'étendant longitudinalement.

12. Vêtement selon l'une quelconque des revendications précédentes, dans lequel la ou chaque marge latérale (80) a une largeur supérieure à 3 mm et inférieure à 75 mm, de préférence comprise entre environ 15 mm et environ 45 mm, et mieux entre environ 25 mm et environ 35 mm.

13. Vêtement selon l'une quelconque des revendications précédentes, dans lequel le composite absorbant comprend des marges latérales opposées s'étendant transversalement vers l'extérieur sur chaque côté dudit composite absorbant (50) et se terminant en bords libres opposés, les marges latérales ayant une largeur combinée qui est d'au moins 3% sans excéder environ 75 % de la largeur transversale totale du composite absorbant, mieux comprise entre 15% et 50% de la largeur transversale totale du composite absorbant, et mieux encore entre environ 25% et 35% de la largeur transversale totale du composite absorbant.

FIG.1

FIG.2

FIG.3

FIG.4

# FIG.5

EP 1 392 212 B2

FIG.6

FIG.7

FIG.8

FIG.9

EP 1 392 212 B2

FIG.10

FIG.11

FIG.12

# FIG.13

FIG.14

FIG.15

FIG.16

FIG.17

FIG.18

FIG.19

FIG.20

# FIG.21

**FIG. 22**

**FIG. 23**

**FIG. 24**

# FIG.25

FIG.26

FIG.27

FIG.28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9614815 A
- US 6132410 A
- US P4699823 A, Kellenberger
- US 3901236 A
- US 4076663 A
- US 4286082 A, Tsubakimoto